# EUROPEAN PATENT SPECIFICATION

(11) **EP 3 731 937 B1**
(45) Date of publication and mention of the grant of the patent: **31.05.2023**
(21) Application number: 17825888.5
(22) Date of filing: 29.12.2017
(51) Int. Cl.: A61K 8/04, A61K 8/34, A61Q 11/00, A61K 8/73, A61K 8/19, A61K 8/21

(54) **DENTIFRICE COMPOSITION**
ZAHNPASTAZUBEREITUNG
COMPOSITION DE DENTIFRICE

(43) Date of publication of application: 04.11.2020
(73) Proprietor: GABA International Holding GmbH, 4106 Therwil (CH)
(72) Inventor: FONTERS, Jessie, 1782 Lossy (CH); MATUR, Turan, 4102 Binningen (CH); BRUNELLA, Andre, 4143 Dornach (CH); HUBER, Norbert, 4058 Basel (CH)
(74) Representative: Sonnenhauser, Thomas Martin
(86) International application number: PCT/EP2017/084834
(87) International publication number: WO 2019/129365

(56) References cited:
- JP-A- 2016 074 723
- US-A1- 2013 323 186
- ARNAUD T M S ET AL: "Chitosan effect on dental enamel de-remineralization: An in vitro evaluation", JOURNAL OF DENTISTRY, ELSEVIER, AMSTERDAM, NL, vol. 38, no. 11, 1 November 2010 (2010-11-01), pages 848-852, XP027355317, ISSN: 0300-5712 [retrieved on 2010-06-19]
- GHORMADE V ET AL: "Can fungi compete with marine sources for chitosan production?", INTERNATIONAL JOURNAL OF BIOLOGICAL MACROMOLECULES, ELSEVIER BV, NL, vol. 104, 29 January 2017 (2017-01-29), pages 1415-1421, XP085173021, ISSN: 0141-8130, DOI: 10.1016/J.IJBIOMAC.2017.01.112

## Description

### FIELD OF THE DISCLOSURE

The present invention relates to dentifrices for use in treating or preventing erosive tooth demineralization in acidic media, brought about by food acids or endogenous acids such as gastric juice.

### BACKGROUND

There are three major sources for acids, which can cause tooth demineralization. The first source are the acids generated by cariogenic oral bacteria from food debris. These acids are carboxylic acids derived from the carbohydrates of the food debris that are metabolized by the oral bacteria. Such acids are rather weak, but act for extended periods on the teeth. The second source is the exogenous food acids that are present in the foodstuffs themselves, in particular in fruits, fruit juices or in artificial soft drinks, or in salad dressings. The third source are endogenous acids, in particular hydrochloric acid-containing gastric juice, which may come into contact with the teeth upon vomiting, such as in bulimia patients, or in reflux disease patients. These latter two types of acids are rather strong but act only for short times on the teeth. Tooth demineralization caused by the latter two types of acids is termed "erosive tooth demineralization" and is not related to cariogenic oral bacteria. Since acid-containing soft drinks have enjoyed a rising popularity among consumers in the past time the problem of erosive tooth demineralization by food acids has become more acute, and a marked percentage of the overall population is nowadays afflicted by it. Similarly, a rising number of (mainly female) patients are subject to bulimia. Erosive tooth demineralization is not noticed by the afflicted subject for quite a long time, and the pathological condition is thus often only diagnosed at a very late stage. Since erosive tooth demineralization is considered irreversible (in contrast to tooth demineralization caused by cariogenic bacteria) it is essential that it be prevented from happening in the first place, or if it has already taken place, that it be prevented from proceeding further or that its progression be slowed down.

Fluorides are customarily used in oral care products such as toothpaste, dental gel or mouthwash. It has been known for a long time that fluoride ion, optionally in combination with stannous ions, such as in the form of stannous fluoride, is beneficial in preventing erosive tooth demineralization.

Chitosan has occasionally been used or studied in oral care. WO 12/110106 GB describes oral care compositions containing chitosan and tin ions, fluoride ions and an abrasive for treatment and prevention routes against erosive toot demineralization caused by strong food acids or by strong endogenous acids such as gastric juice. GB 2132889A describes oral care products containing chitin derivatives such as chitosan, and discloses that chitin or chitosan may act as a cure or prophylaxis in case of dental caries, periodontoclasia and halitosis, and that in a dentifrice chitosan salts may mask the taste of a silica abrasive. WO 02/17868A describes oral and dental hygiene agents containing chitosan microcapsules, the microcapsules being loaded with an active agent which may be, among others, stannous fluoride. Its compositions are said to have protective effect against caries, periodontitis and plaque, and to have anti-inflammatory effect. WO 03/042251 A discloses compositions, such as oral care compositions, comprising chitosan in the form of nano-sized fibers and which also may contain a fluoride source. These compositions are said to improve general gum and teeth health, to be suitable for treatment of halitosis and gingivitis, to reduce staining of the teeth, to provide anti-caries, anti-plaque and anti-calculus benefits, to inhibit cariogenic bacteria, and to inhibit hydrogen sulphide and volatile odiferous organosulphide compounds produced by salivary microorganisms. For the chitosan itself it is stated that it has film-forming and pH-buffering capabilities. JP 2006/241 122A discloses compositions, which may be oral care compositions, which comprise glucosamine and/or chitosan oligosaccharide, and a remineralization promotion constituent containing a fluorine ion source. The "remineralization" is in the case of carious lesions produced by streptococcus mutans. WO 2008/121518A discloses polymeric microcapsules, which may preferably be chitosan microcapsules, and which may be used in dentifrices which may contain a fluoride source. The capsules also contain a quaternary ammonium salt. The compositions are said to be antimicrobial. Recently a toothpaste called "Chitodent" has appeared on the German market. According to its advertisement it contains chitin, chitosan and silver ions, but is devoid of fluoride. Stamford Arnaud TM et al. J Dent 38(2010)848-852 studied the remineralizing effect of chitosan in human tooth samples which had been demineralized with acetate buffers of pH 4.0 and 4.8, which is a model for caries-related demineralization. Ganss C, Schlueter N. Quintessenz 61 (2010)1203-1210 discusses prospective new agents for the indication of erosive tooth demineralization and mentions chitosan but states that "proof of activity so far is not available". In a poster by Neutard et al. presented at the 57th congress of the European Organization for Caries Research (ORCA, Montpellier, France, July 2010), activities of some fluoride-containing toothpastes and some "special free fluoride-free toothpastes" (among which was the above mentioned Chitodent) in the prevention of erosive tooth demineralization were determined. The authors concluded that "the fluoride-free preparations had no significant effect" and that "the special formulations were not superior or even less effective compared to conventional products".

The present application seeks to provide improved treatment and prevention routes against erosive tooth demineralization caused by strong food acids or strong endogenous acids such as gastric juice.

### BRIEF SUMMARY

The task set is solved by a dentifrice comprising non-crustacean chitosan or a pharmaceutically acceptable acid addition salt thereof, fluoride ions as defined in the claims and an abrasive, for use against erosive tooth demineralization, which shows a surprising improvement in the extent of enamel tissue retention after repeated acid challenges when compared against a comparable toothpaste which contains crustacean chitosan.

The invention can be used in the form of a kit comprising:
a) A dentifrice comprising a combination of non-crustacean chitosan or pharmaceutically acceptable acid addition salt thereof, fluoride ions as defined in the claims and an abrasive; and
b 1) a container containing the dentifrice and bearing human-readable indications disclosing that the dentifrice is for use against erosive tooth demineralization, or
b2) a package containing a container, the container comprising the dentifrice, and the package bearing human-readable indications disclosing that the dentifrice is for use against erosive tooth demineralization, or
b3) a package containing a container and a leaflet, the container comprising the dentifrice, and the leaflet bearing human-readable indications disclosing that the dentifrice is for use against erosive tooth demineralization.

The invention also refers to a dentifrice for use in a method for the prevention of erosive tooth demineralization or for the treatment of teeth affected by erosive tooth demineralization in a subject in need of such prevention or treatment, wherein the method comprises bringing the subject's teeth in contact with a dentifrice comprising non-crustacean chitosan or a pharmaceutically acceptable acid addition salt thereof, fluoride ions and an abrasive.

Oral care articles containing fluoride ions as an agent against erosive tooth demineralization, and non-crustacean chitosan or a pharmaceutically acceptable acid addition salt thereof, as a combination for the simultaneous, separate or successive administration in the prevention or treatment of erosive tooth demineralization, with the provisos that the oral care articles contain a dentifrice comprising an abrasive, and that either the fluoride ions or the chitosan or pharmaceutically acceptable acid addition salt thereof are contained in the dentifrice.

A toothpaste comprising a liquid phase, 200 to 2000 ppm, preferably 1000 to 1800 ppm fluoride ions, 0.05 to 1 % of non-crustacean chitosan or a pharmaceutically acceptable acid addition salt thereof, 3000 to 4000 ppm tin dissolved in the liquid phase, 17 to 27% of glycerol, 17 to 27 % of sorbitol and 0.3 to 1% of gluconate, all % being based on the toothpaste; and one or more abrasives in a total amount such as to impart the toothpaste a PCR value of at least 50.

Preferred embodiments of all these objects are as in the respective dependent claims and as outline hereinafter.

Further areas of applicability of the present invention will become apparent from the detailed description provided hereinafter. It should be understood that the specific examples, while indicating the preferred embodiment of the invention, are intended for purposes of illustration only and are not intended to limit the scope of the invention.

### DETAILED DESCRIPTION

The invention refers to a dentifrice comprising: a source of stannous ions; a source of fluoride ions as defined in the claims; a non-crustacean derived chitosan, or pharmaceutically acceptable acid addition salt thereof; and an abrasive system, wherein said chitosan has a molecular weight of 15 to 40 kDa, and is present in an amount of 0.4 to 1.5 weight percent based on the total composition. In an embodiment, a dentifrice is provided wherein the amount of abrasive is from 12 to 30 percent by weight based on the total composition.

In an embodiment, a dentifrice is provided, wherein the non-crustacean derived chitosan is fungus-derived chitosan.

In an embodiment, a dentifrice is provided , wherein the non-crustacean derived chitosan has a molecular weight of 20 to 35 kDa.

In an embodiment, a dentifrice is provided wherein the toothpaste comprises an abrasive system in an amount effective to provide a PCR value of at least 50.

In an embodiment, a dentifrice is provided wherein the dentifrice comprises: 3000 to 4000 ppm of stannous ions; 200 to 2000 ppm, preferably 1000 to 1800 ppm fluoride ions; 0.4 to 1.5 weight percent of non-crustacean derived chitosan, or a pharmaceutically acceptable acid addition salt thereof; 17 to 27 weight percent of glycerol; 2 to 15 weight percent of sorbitol; and an abrasive system in an amount effective to provide a PCR value of at least 50;wherein the weight percent is based on the total weight of the dentifrice.

In an embodiment, a dentifrice is provided comprising: 1300 to 1500 ppm of fluoride ions; 0.5 to 1 weight percent of non-crustacean derived chitosan, or a pharmaceutically acceptable acid addition salt thereof; 3300 to 3700 ppm of stannous ions; 20 to 24 weight percent of glycerol; and 3 to 6 weight percent of sorbitol; wherein the weight percent is based on the total weight of the dentifrice.

In an embodiment, a dentifrice is provided wherein the composition comprises a liquid phase.

In an embodiment, a dentifrice is provided wherein the stannous ions are in the liquid phase.

In an embodiment, a dentifrice is provided wherein the fluoride ions are in the liquid phase.

In an embodiment, a dentifrice is provided wherein the non-crustacean chitosan is in the liquid phase.

In an embodiment, the dentifrice is provided, wherein at least one of the stannous ions, the fluoride ions and the non-crustacean chitosan is dissolved in the liquid phase.

The dentifrice of the invention is provided for use in a method for treating or preventing tooth demineralization in a subject in need thereof, comprising: applying a dentifrice comprising: a source of stannous ions; a source of fluoride ions as defined in the claims; a non-crustacean derived chitosan, or pharmaceutically acceptable acid addition salt thereof; and an abrasive system, wherein said chitosan has a molecular weight of 15 to 40 kDa, and is present in an amount of 0.4 to 1.5 weight percent based on the total composition, to an oral cavity surface of said subject.

In an embodiment, the method is for treating or preventing tooth demineralization in a subject in need thereof, comprising applying a dentifrice according to the invention to an oral cavity surface of said subject;
The dentifrice of the invention can be used as a kit comprising:
a) a dentifrice comprising a combination of fungus-derived chitosan or pharmaceutically acceptable acid addition salt thereof, stannous ions, fluoride ions as defined in the claims and an abrasive; and
b1) a container containing the dentifrice and bearing human-readable indications disclosing that the dentifrice is for use against erosive tooth demineralization, or
b2) a package containing a container, the container comprising the dentifrice, and the package bearing human-readable indications disclosing that the dentifrice is for use against erosive tooth demineralization, or
b3) a package containing a container and a leaflet, the container comprising the dentifrice, and the leaflet bearing human-readable indications disclosing that the dentifrice is for use against erosive tooth demineralization.

The indications disclose that the dentifrice is for the prevention of erosive tooth demineralization or for the treatment of teeth affected by erosive tooth demineralization.

The dentifrice can be a toothpaste comprising a liquid phase, and wherein at least one of the fungus-derived chitosan or pharmaceutically acceptable acid addition salt thereof, the stannous ions and the fluoride ions are in the liquid phase.

The toothpaste may furthermore comprise tin dissolved in the liquid phase.

The toothpaste may comprise one or more abrasives in a total amount such as to impart the toothpaste a PCR value of at least 50; and wherein the indications disclose that the toothpaste is for the treatment of teeth affected by erosive tooth demineralization.

The method for the prevention of erosive tooth demineralization or for the treatment of teeth affected by erosive tooth demineralization in a subject in need of such prevention or treatment, comprises bringing the subject's teeth in contact with a dentifrice comprising fungus-derived chitosan or a pharmaceutically acceptable acid addition salt thereof, stannous ions, fluoride ions as defined in the claims and an abrasive.

In an embodiment, the dentifrice is a toothpaste comprising a liquid phase, and wherein the fungus-derived chitosan or pharmaceutically acceptable acid addition salt thereof and the fluoride ions are dissolved in the liquid phase.

In an embodiment, the toothpaste furthermore comprises tin dissolved in the liquid phase;
In an embodiment, the toothpaste comprises one or more abrasives in a total amount such as to impart the toothpaste a PCR value of at least 50; and wherein the subject's teeth are affected by erosive tooth demineralization.

An embodiment refers to an improved dentifrice comprising a source of stannous ions, a source of fluoride ions as defined in the claims, chitosan or pharmaceutically acceptable acid addition salt thereof, and an abrasive, for use against erosive tooth demineralization, wherein said chitosan is fungus-derived chitin, having a molecular weight of 15 to 40 kDa, and is present in an amount of 0.4 to 1.5 weight percent based on the total composition, and wherein said dentifrice provides a reduction in erosive demineralization versus dentifrice containing crustacean-derived chitosan at equivalent weight percent chitosan and equivalent fluoride content.

In an embodiment, a dentifrice as defined in the claims is provided for use in the prevention of erosive tooth demineralization or the treatment of teeth affected by erosive tooth demineralization and the amount of abrasive is from 12 to 30 percent by weight based on the total composition.

In an embodiment, the dentifrice is a toothpaste comprising a liquid phase, and wherein the fungus-derived chitosan or pharmaceutically acceptable acid addition salt thereof and the fluoride ions are dissolved in the liquid phase.

In an embodiment, the fungus-derived chitosan has a molecular weight of 20 to 35 kDa.

In an embodiment, stannous ions are present in the liquid phase.

In an embodiment, the dentifrice is for use in the treatment of teeth affected by erosive tooth demineralization or prevention of erosive tooth demineralization and the toothpaste comprises one or more abrasives in a total amount such as to impart the toothpaste a PCR value of at least 50.
4.6. In an embodiment, the dentifrice comprises a liquid phase, 200 to 2000 ppm, preferably 1000 to 1800 ppm fluoride ions, 0.4 to 1.5% of fungus-derived chitosan or a pharmaceutically acceptable acid addition salt thereof, 3000 to 4000 ppm tin dissolved in the liquid phase, 17 to 27% of glycerol, 2 to 15% of sorbitol, all % being based on the toothpaste; and one or more abrasives in a total amount such as to impart the toothpaste a PCR value of at least 50;
In an embodiment, the dentifrice comprises 1300 to 1500 ppm fluoride ions as defined in the claims, 0.5 to 1% of fungus-derived chitosan or a pharmaceutically acceptable acid addition salt thereof, 3300 to 3700 ppm tin dissolved in the liquid phase, 20 to 24% of glycerol, 3 to 6% of sorbitol, all % being based on the toothpaste.

The present invention comprises, as a first component, non-crustacean chitosan, such as fungus-derived chitosan or a pharmaceutically acceptable acid addition salt thereof. An example of fungus-derived chitosan is Chitosan KiOnutrime-CsG from fungal origin (MW ca. 30 kDa, KitoZyme/Belgium, source organism *Aspergillus niger).* The chitosan may preferably have a degree of deacetylation (DDA) of 70% to 100%. The DDA (in percent) of a chitosan sample may be obtained by titration. The chitosan is preferably in a form where its deacetylated amino groups are protonated with a pharmaceutically acceptable acid, to form a pharmaceutically acceptable acid addition salt of the fungus-derived chitosan. The protonation degree, i.e. the mole fraction of deacetylated amino groups that are protonated, is preferably in the range of 80 mole% to 99 mole%, more preferably in the range of 90 mole% to 95 mole%. As pharmaceutically acceptable acids that can be used to form the pharmaceutically acceptable acid addition salt thereof may be mentioned mineral hydrohalic acids, such as hydrochloric or hydrofluoric acids; mineral oxo acids, such as sulfuric, phosphoric, or nitric acids; or organic carboxylic acids. The fungus-derived chitosan or pharmaceutically acceptable acid addition salt thereof preferably has an average molecular weight in the range of 5 000 to 50 000 Daltons, more preferably in the range of 15 000 to 40 000 Daltons, particularly preferably in the range of 20 000 to 35 000 Daltons. This average molecular weight and molecular weight distribution may be determined in a known manner by gel permeation chromatography using e.g. N-acetyl-D-glucosamine oligomer and pullulan retention time standards, or by using a multi angle laser light scattering (MALLS) detector.

Preferably, the chitosan is not further chemically modified by additional functional groups such as hydrophilic or charged side groups, N-carboxymethyl, N,N-dicarboxymethyl, N-methylene phosphonic, N-methyl, N-monocarboxybutyl, N,N-dicarboxybutyl, 5-methylpyrrolidinone and N-trimethyl This is referred to as an unmodified chitosan.

The invention requires, as a second component, fluoride ions. The source of fluoride ions comprises sodium fluoride, and amine fluoride.

These two components are used according to the invention in such a form that at least one of the two is contained in a dentifrice containing an abrasive. As "dentifrice containing an abrasive" are understood in particular toothpastes and dental powders; by such term dental gels are excluded, the latter being devoid of abrasives. The abrasives that can be used themselves are conventional. Preferred abrasives are inorganic abrasives, such as precipitated silicas, aluminas, insoluble carbonates (e.g. calcium carbonate, calcium phosphate, calcium pyrophosphate), zeolites or stannous pyrophosphate; or organic abrasives such as polyethylene, polyvinyl chloride, polystyrene, polycarbonate, copolymers from (meth)acrylates and other olefinic monomers, polyamides, urea-formaldehyde resins, melamine-formaldehyde resins, phenol-formaldehyde resins, cured, pulverized epoxy resins or polyesters. A mixture of these abrasives may also be used. Preferred is an abrasive mixture of hydrated silica(s) with a small amount, such as 5 to 20 % based on the total of the hydrated silica(s), of alumina. Toothpastes suitable for the uses of the invention may also comprise essentially non-abrasive silicas, having only a thickening effect on the toothpaste formulations.

The main distinction between a toothpaste and a dental powder is that the former also comprises a liquid phase, whereas the latter is a dry powder which is slurried in the oral cavity upon use with saliva. The use according to the invention in the form of a toothpaste is preferred. In the latter case it is preferred that anyone of these components that is contained in the toothpaste be dissolved in its liquid phase.

The dentifrices, articles or kits of the invention are for use against, and are efficacious in, the treatment or prevention, particularly the prevention of erosive tooth demineralization caused by food acids (i.e. acids originating from foods) or by endogenous acids such as gastric juice (hydrochloric acid). As "food acids" are considered in the context of the present application such acids with a pKa value (or first pKa value, if multibasic) of 5.0 or less. Examples therefore are citric acids (e.g. from fruits), tartaric acid (e.g. from wine), oxalic acid (e.g. from rhubarb), phosphoric acid (e.g. from soft drinks), hydrated sulphur dioxide (e.g. from wine), and amino acids.

The chitosan or a pharmaceutically acceptable acid addition salt thereof and the fluoride ions may either be contained in a single dentifrice, containing them as a "fixed" combination. They may on the other hand be included into separate oral care formulations, wherein one formulation contains the chitosan and/or pharmaceutically acceptable salt thereof and the other oral care formulation contains the fluoride, provided that at least one of the two agents is included into a dentifrice containing an abrasive. Such oral care formulation kits, also designated in the following as "oral care articles" or, for short, "articles", may be intended for either simultaneous administration, i.e. the two formulations are used by one and the same subject at the same time, or for separate administration, i.e. the two formulations are used independently by one and the same subject, but not according to a specified dosage regime, or for successive administration, i.e. the two formulations are used by one and the same subject one after the other, in particular one immediately after the other, in particular according to a specified dosage regime.

An example for such an article is a kit containing, as a first oral care formulation, a toothpaste comprising an abrasive and comprising a liquid, in particular aqueous phase, wherein in that liquid phase chitosan or a pharmaceutically acceptable acid addition salt thereof is dissolved; and containing, as a second formulation, a mouthrinse consisting of a liquid, preferably aqueous phase and comprising dissolved fluoride ions. In this exemplary kit, it may also be possible to include the fluoride into the toothpaste, by dissolving in its liquid phase, and the chitosan or pharmaceutically acid addition salt thereof into the mouthrinse. In either of these two variants, optionally and preferably dissolved tin as described hereinbefore may be present, the tin also being preferably dissolved in the mouthrinse or the liquid phase of the toothpaste.

Said articles are preferably intended for separate or sequential use of its two formulations, according to a dosage regime similar to conventional such toothpaste/mouthrinse articles.

The content of dissolved chitosan and/or its pharmaceutically acceptable acid addition salt in the dentifrice (when it is a dentifrice containing both active agents as a "fixed combination) or in the oral care formulation containing the chitosan or salt thereof (in the case of articles having two or more oral care formulations) is firstly chosen at least sufficiently high such as to observe a statistically significantly higher activity, in combination with the fluoride ions, than is observed in the same experimental setup, but with fluoride ions alone. As "statistically significant" is understood if a two-sided Student's T-test, with a confidence limit of 5%, detects such significant difference in activity between the combination fluoride ions plus chitosan or salt thereof, and fluoride ions alone. Such statistically significant difference is indicative of a synergistic action between fluoride ions and the chitosan or salt thereof. The content of dissolved chitosan and/or its pharmaceutically acceptable acid addition salt is secondly chosen not higher than as to impart the toothpaste (or the liquid phase of the oral care formulation containing the chitosan or salt thereof, in the case of articles having two or more oral care formulations) an overall dynamic viscosity of at the most 1500 Pa*s. The skilled person is well aware on how to choose the proper amount, molecular weight and DDA of the fungus-derived chitosan or pharmaceutically acceptable acid addition salt thereof, in order to achieve, depending on the pH, the ionic strength and any other viscosity-affecting components of the toothpaste, the desired dynamic viscosity thereof. Typically the amount of fungus-derived chitosan or pharmaceutically acceptable acid addition salt thereof is preferably 0.2 to 5%, more preferably 0.4 to 1.5%, still more preferably 0.5 to 1.0%, by weight, based on the weight of the dentifrice, toothpaste or formulation of the article in question.

The fluoride ion content of the dentifrice (when it is a dentifrice containing both active agents as a "fixed" combination) or in the oral care formulation containing fluoride (in the case of articles having two or more oral care formulations) is preferably from 200 to 2000 ppm, based on the dentifrice, or based on the formulation in question. If in a dentifrice/mouthrinse kit the dentifrice is a toothpaste and the fluoride ions are comprised in that toothpaste, then the fluoride ion concentration is more preferably from 1000 to 1600 ppm, most preferably from 1300 to 1500 ppm, based on the toothpaste, wherein the fluoride ions are preferably dissolved in the liquid phase of the toothpaste. The fluoride ion content may be determined potentiometrically using a fluorideselective electrode (see example 9).

The fluoride ions and the chitosan or pharmaceutically acceptable acid addition salt thereof are preferably dissolved in a liquid phase of a toothpaste. The liquid phase is preferably at least partially aqueous. Accordingly, the liquid phase may preferably comprise about 10% to about 90%, more preferably about 25% to about 75%, based on the liquid phase, of water. The liquid phase may have a pH which is physiologically acceptable and which preferably serves to fully dissolve the entire amount of chitosan. Such pH may typically be in the range of about 3.0 to about 6.0, preferably about 4.0 to about 5.0, more preferably about 4.3 to about 4.6. If necessary the pH of the liquid phase may be adjusted to the desired value by adding acid (such as hydrochloric acid) or base (such as sodium hydroxide).

The toothpastes and any other oral care formulations within articles are preferably devoid of silver, meaning that they comprise preferably less than 0.05%, more preferably less than 0.001%, based on the composition, of silver.

When any dentifrice is a toothpaste having a liquid phase (whether containing both fluoride ions and chitosan or pharmaceutically acceptable salt thererof as a "fixed" combination, or containing only one of these two and forming part of an articles having two or more oral care formulations) then it furthermore preferably also comprises tin dissolved in that liquid phase. The term "dissolved tin", as used herein, is intended to encompass all ionic or non-ionic tin species in the formal oxidation states +II and/or +IV and being dissolved in the liquid phase. Examples of such dissolved tin species are hydrated stannous ions, stannous hydroxide, soluble ionic or nonionic complexes of stannous and/or stannic ions with ligands, such as with an optionally also present dissolved C₍₃₋₆₎ sugar alcohol and/or the anionic conjugate base of an optionally also present dissolved organic acid as ligands, and ionic hydroxo complexes of stannous and/or stannic ions. Preferably 60 mole% or more, more preferably 75 mole% or more of the content of dissolved tin [Sn] is tin in the formal oxidation state +II. For a toothpaste contained within an article it is preferably 3000 to 4000 ppm, more preferably 3300 to 3700 ppm. The total content of dissolved tin may be determined using X-ray fluorescence. The content of dissolved tin in the formal oxidation state +II may be determined potentiometrically. The dissolved tin may preferably be derived from a pharmaceutically acceptable stannous ion salt. Examples are stannous chloride, stannous fluoride, stannous hydroxide, stannous sulphate, with stannous chloride being preferred.

In the dentifrices of the instant invention, the fluoride ion source comprises sodium fluoride, and amine fluoride.

Preferably the fluoride is used as a mixture of sodium fluoride and amine fluoride such that the amount ratio fluoride ions derived from sodium fluoride : fluoride ions derived from amine fluoride is in the range of 0.7: 1 to 1.4 : 1, more preferably 0.9: 1 to 1.1 : 1.

In all embodiments the amine fluoride preferably contains ammonium cations of the formula R-NH⁺Rₐ-[(CH₂)ᵤ-NH⁺R_{b}]ᵥ-R_{c}, wherein R is a saturated or unsaturated straight-chain hydrocarbon residue of 10 to 20 carbon atoms, v is an integer from 0 to 1, u is an integer from 2 to 3 and Rₐ, R_{b} and R_{c} are independently selected from hydrogen and -CH₂CH₂OH. The residue R can have even or odd-numbered chain length, residues R having an even-numbered chain length are preferred with regard to physiological acceptability. The residues may be preferably monounsaturated. Examples of saturated hydrocarbon residues having an even-numbered chain length are decyl, dodecyl (lauryl), tetradecyl (myristyl), hexadecyl (cetyl, palmityl), octadecyl (stearyl) and eicosanyl. Examples of unsaturated residues having an even-numbered chain length are 9-cis-octadecen-1-yl (oleyl), 9-trans-octadecen-1-yl (elaidyl), cis,cis-9, 12-octadecadien-1-yl (linolyl), cis,cis,cis-9, 12, 15-octadecatrien-1-yl (linolenyl) or 9-cis-eicosaen-1-yl (gadolyl). More preferred are C₁₈ alkyl or C₁₈ alkenyl, in particular 9-cis-octadecen-1 -yl (oleyl). The most preferred cation in all embodiments of the invention is with R = oleyl, Rₐ = R_{b} - R₀ ⁼ -CH₂CH₂OH, v = 1 and u = 3, i.e. wherein the amine fluoride is olaffur (N-(9-cis-octadecen-1-yl)-N,N'N'-tris(hydroxyethyl)-1 ,3-diaminopropane dihydrofluoride). The amount of ammonium cations may be determined according to example 10 or 11.

The toothpastes of the invention may furthermore comprise one or more C₍₃₋₆₎ sugar alcohols. The term "C₍₃₋₆₎ sugar alcohol" is intended to encompass all polyhydric alcohols with a total carbon atom number n of 3 to 6 and a molecular formula of CₙH₍₂ₙ₊₂₎Oₙ. Preferably these sugar alcohols are acyclic and unbranched. Examples of the C₍₃₋₅₎ sugar alcohol are glycerol, erythritol, threitol, arabitol, xylitol, ribitol, sorbitol and mannitol. For a toothpaste a mixture of glycerol and sorbitol is preferred. Glycerol may be present in amounts of 1 to 30%, 17 to 27%, 20 to 24% based on the toothpaste. Sorbitol may be present in amounts of 1 to 30%, 2 to 15%, 3 to 6% based on the toothpaste. The one or more C₍₃₋₆₎ sugar alcohols are preferably dissolved in the liquid phase of the toothpaste.

The toothpastes of the invention may furthermore comprise an organic acid and/or salt thereof, either as part of a buffering system intended to achieve the above mentioned physiologically acceptable pH of the liquid phase, or as a complexing agent for dissolved tin species, if present. The organic acid, if present, is preferably a carboxylic acid. It is preferably dissolved in the liquid phase of the toothpaste. The term "dissolved" implies here that the acid be dissolved either as the free acid or as a pharmaceutically acceptable salt of its anionic conjugate base (whichever may be the case) in the liquid phase. Preferred subgroups of organic acids are edible di- or tricarboxylic acids with 4 to 6 carbon atoms including the carboxylate carbon atoms, such as succinic, tartaric, citric, malic, fumaric and adipic acids; or edible a-hydroxy C₍₂₋₆₎carboxylic acids such as glycolic, lactic, citric, tartaric or gluconic acids.

If the organic acid is dissolved in the form of a pharmaceutically acceptable salt then the counter cation may be a metal cation, such as from an alkaline metal (such as sodium or potassium), from an earth alkaline metal (such as magnesium or calcium), or from zinc. When organic acid is present, then its content is preferably in the range of 0.01 to 10%, preferably 0.05 to 5%, based on the dentifrice, whereby the upper limit may be given by the solubility of its conjugate base salt in the liquid phase at physiologically acceptable pH. The total content of organic acids may be determined by acidifying a known aliquot of the oral care composition to about pH 0, extracting the free organic acids with an organic solvent such as ether, and analyzing the extract by calibrated GC using the silyl esters derivates of the acids. More preferably the toothpastes of the invention contain 0.3 to 1.0% of gluconic acid or of a salt thereof (i.e. gluconate).

Further optional components in the dentifrices, in particular the toothpastes, may be for instance:
- Flavorings and cooling flavors, such as coumarin, vanillin, ethereal oils (such as peppermint oil, spearmint oil, aniseed oil, menthol, anethol or citrus oil) or other essences (such as apple, eucalyptus or spearmint essence). These flavorings may be present in 0% to 0.5%, preferably 0.03% to 0.3%, based on the dentifrice.
- Sweeteners, in particular artificial sweeteners such as saccharin, acesulfam, neotam, cyclamate or sucralose; natural high-intensity sweeteners such as thaumatin, stevioside or glycyrrhizin; or sugar alcohols different from the C₍₃₋₅₎ sugar alcohol, such as sorbitol, xylitol, maltitol or mannitol. These may be present in amounts of 0% to 0.2%, preferably 0.005% to 0.1%, based on the dentifrice.
- Antibacterials and/or preservatives, such as chlorhexidine, triclosan, quaternary ammonium compounds (such as benzalkonium chloride) or parabens (such as methyl or propyl paraben). The amount of antimicrobial agent is typically from 0 to about 0.5%, preferably 0.05 to 0.1%, based on the dentifrice.
- Emulsifiers or solubilizes, mainly in connection with abovementioned flavorings and/or antibacterials, which often are of low solubility in aqueous media. Examples of such emulsifiers are neutral surfactants (such as polyoxyethylene hydrogenated castor oil or fatty acids of sugars), anionic surfactants (such as sodium lauryl sulphate), cationic surfactants (such as the ammonium cations of formula (I)) or zwitterionic surfactants. These surfactants or solubilizes may be pre-sent in amounts of typically 0% to 2%, preferably 0.2% to 1.5%, based on the dentifrice.
- Thixotropic agents, such as soluble grades of hydroxypropylmethylcellulose, hydroxyethylcellulose or mucins, in an amount effective to impart the dentifrice a thixotropic behavior.
- Stabilizers, such as polyvinylpyrrolidone.

The dentifrices or articles are intended for use against erosive tooth demineralization. For this purpose they are suitably provided as a kit containing the composition and human-readable indications disclosing to the subject using the composition that the composition is for use, or efficacious, against erosive tooth demineralization. These indications may be directly printed on the container comprising the dentifrice (such as a toothpaste tube), or they may be printed on a label wrapped or adhered onto the container. They may also be printed on a package, such as a cardboard box, enclosing the container. Finally they may be printed on a leaflet (a package insert), to be included into the kit.

The dentifrices of the invention may be used to prevent or treat erosive tooth demineralization in a subject in need of such prevention or treatment. As "treatment" is preferably understood here the so-called "secondary prevention", which is a treatment on subjects exhibiting early or intermediate stages of erosive tooth demineralization, in order to slow down a further progression of the demineralization.

Patients in need of prevention are subjects having at least one of the following habits or conditions 1)-5):
1) They regularly consume acidic foods, in particular acidic beverages such as soft drinks;
2) they suffer from reflux disease or bulimia,
3) they clean their teeth to an extent to remove essentially all of the salivary pellicle on their tooth surfaces;
4) they have an anomaly in the chemical properties of their saliva, particularly such as below-normal levels of calcium and/or phosphate, or below-normal buffering capacity;
5) they exhibit insufficient saliva production (xerostomia patients).
Particularly patients in need of prevention are understood as subjects having 1) in combination with one of 3) to 5), or subjects having 2) in combination with one of 3) to 5). Patients in need of treatment, particularly in need of the abovementioned secondary prevention, are subjects having at least one of the above l)-5), or having 1) in combination with one of 3) to 5), or having 2) in combination with one of 3) to 5); and showing the signs of early or intermediate stages of erosive tooth demineralization.

As used throughout, ranges are used as shorthand for describing each and every value that is within the range including its boundary values. Any value within the range can be selected as the terminus of the range.

Unless otherwise specified, all percentages and amounts expressed herein and elsewhere in the specification should be understood to refer to percentages by weight. The amounts given are based on the active weight of the material,

The invention will now be further explained by the following non-limiting examples. In the examples "AmF" or "AmF 297" denotes the amine fluoride.

### EXAMPLES

Example 1: In situ demineralization study on enamel with toothpastes containing a combination of the invention and with reference examples.

The study products were five toothpastes according to the following Table 1:

| Table 1. | | Comparative | Ex. 1 | Ex. 2 | Ex. 3** | Ex. 4** | Ex. 5 |
|---|---|---|---|---|---|---|---|
| | Glycerine | 22.0 | 22.0 | 22.0 | 22.0 | 22.0 | 22.0 |
| | Sorbitol 70% | 8.0 | 8.0 | 8.0 | 8.0 | 8.0 | 8.0 |
| | Crustacean Chitosan | 0.50 | 0.0 | 0.0 | 0.0 | 0.0 | 0.0 |
| | Non-crustacean Chitosan | 0.0 | 0.5 | 1.0 | 0.5 | 0.5 | 0.5 |
| | SnCl2 (1000g/l) | 1.0 | 1.0 | 1.0 | 1.0 | 1.0 | 1.0 |
| | Hydrated Silica | 22.0 | 22.0 | 22.0 | 22.0 | 17.0 | 27.0 |
| | Hydroxyethyl cellulose | 1.6 | 1.6 | 1.60 | 1.60 | 1.85 | 1.45 |
| | Aluminum Oxide | 0.5 | 0.0 | 0.0 | 0.0 | 0.0 | 0.0 |
| | Pigment | 1.0 | 2.0 | 2.0 | 2.0 | 2.0 | 1.0 |
| | Sweetener | 0.2 | 0.2 | 0.2 | 0.2 | 0.2 | 0.2 |
| | Buffering Agent | 0.75 | 0.75 | 0.75 | 0.75 | 0.75 | 0.75 |
| | NaF | 0.155 | 0.155 | 0.155 | 0.00 | 0.00 | 0.155 |
| | Surfactant | 3.50 | 3.50 | 3.50 | 0.00 | 0.00 | 3.50 |
| | Flavorant | 1.40 | 1.40 | 1.40 | 1.40 | 1.40 | 1.40 |
| | AmF soln. (1.4% wt.% F) | 5.0 | 5.0 | 5.0 | 10.0 | 10.0 | 5.0 |
| | Water* | q.s. | q.s. | q.s. | q.s. | q.s. | q.s. |

| | | | | | | | |
|---|---|---|---|---|---|---|---|
| ^{∗}The entry q.s. indicates that a sufficient quantity of water was included to total 100%, with pH adjusted to 4.5. ^{∗∗}not claimed | | | | | | | |

The following solutions were used:
Demineralization solution: Erosive demineralization was performed with 0.5% citric acid, prepared by dissolving 5 grams of citric acid monohydrate (Merck, Darmstadt, D) in 1000 ml distilled water. The pH value of the citric acid solution was monitored daily with an ion-selective electrode.

Remineralization solution: The remineralization solution was prepared by first preparing three solutions by dissolving: 0.4 g H3P04 in 40 ml distilled water; 1.5 g KCI in 100 ml distilled water; and 1 g NaHC03 in 100 ml distilled water. These three solutions were combined in a container and a sufficient quantity of distilled water was then added to produce a total solution amount of 600 ml. Then, 0.2 g CaCl2 was dissolved in 100 ml distilled water and the resulting solution was added to the solution described above while stirring. A sufficient quantity of distilled water was then added to his preparation to yield a final volume of 1 liter of remineralization solution. All chemicals are obtained from Merck, Darmstadt, Germany.

Enamel Specimens: Previously impacted human third molars were stored in saturated thymol solution (Thymol, Fluka Chemie AG, Buchs, Switzerland) prior to use. Any remnants of soft tissues were carefully removed and the roots were separated. Approximately 1 mm thick samples were cut from the molar using longitudinal slices into a smooth natural surface. This natural surface was then ground flat by removing about 250-300 µm of material (via diamond grinding and polishing using discs of 30, 15 and 3 µm in succession; Bühler GmbH, Düsseldorf, Germany) until an experimental area of approximately 3 mm × 3 mm was achieved. All cutting, grinding and polishing procedures (Exakt Abrasive Cutting System and Exakt Microgrinder, Exakt-Apparatebau, Norderstedt, Germany) were performed under sufficient water flow. The enamel samples were prepared (n=16 per group) and were cemented onto sample holders suitable for the automated brushing machine using a UV light curing acrylic (Technovit 7230 VLC, Kulzer-Exakt, Wehrheim, Germany). One-half of the flattened area was covered with the acrylic resin and served as a reference area for profilometry. The uncovered experimental area was carefully checked for any contamination. Specimens were stored in 100% humidity until use.

The experimental period was 10 working days. The samples were put on custom made trays enabling the simultaneous transmission into the various solutions. All immersion times were checked with stop watches. All procedures were performed with the samples being in a horizontal position. The erosive demineralization was performed 6×2 min at intervals of 1 hour, in 250 ml 0.5% citric acid (natural pH) on a shaker (Schütteltisch 3006, Gesellschaft für Labortechnik mbH; 35 oscillations/min). Special emphasis was put on the standardised orientation of the sample holders with the long side 90° to the shaking direction of the erosion solution. Afterwards, samples were thoroughly rinsed with tap water for 30 seconds. All samples were immersed in the slurry for 2x2 min after the first and the last demineralization. Immersion was performed in the respective slurry containers of the brushing machine. Within the immersion time, the samples were brushed for 15 sec (ADA reference brush, travel path 6 mm, speed 60 mm/s, load 200 g, zigzag - modus). To assure uniform effects of the brushes, the sample holders were alternately rotated by 180°. After each slurry immersion + brushing, samples were thoroughly rinsed with tap water for 1 min and reinserted in the remineralization solution.

All interventions were performed at room temperature; between interventions samples were stored in the remineralization solution at 25°C in a water bath (Model 1083; Gesellschaft für Labortechnik mbH, Burgwedel, 35/min).

All solutions were freshly prepared at the beginning of each experimental day. During weekends, samples were stored at 100% humidity in the refrigerator (5°C). After the experimental period of 10 days, the samples were stored at 100% humidity in the refrigerator until the profilometry measurements were performed.

Before analysis, the acrylic resin cover was carefully removed from all enamel specimens, and the surfaces were inspected for any acrylic resin remnants or damage. Measurement was performed with an optical device (MicroProf, Fries Research&Technology GmbH, Bergisch-Gladbach, Germany). On each sample, three traces were made at intervals of 0.2 mm, each 2 mm in length (200 pixel, 32 hertz, sensor HO). Traces were interpreted with special software (Mark Ill, Fries Research&Technology GmbH Bergisch-Gladbach, Germany). Parallel regression lines (500 µm in length) were constructed at the ends of the reference and experimental areas. The vertical distance between the regression lines was defined as tissue loss (µm).

The tissue loss value for a given sample was determined by comparing vertical distance between the regression lines of reference area with that of the experimental (treated) area, averaged over the three repetitions of the measurement (profilometer traces) collected per sample.

Statistical procedures were performed using IBM SPSS statistics. Kolmogorov-Smirnov tests revealed that there was no significant deviation of the tissue loss data collected for the different product groups from normal distribution. Levene's test revealed a significant deviation from the homogeneity of variance of the tissue loss data collected for the different groups. Differences in tissue loss between product groups were analyzed using ANOVA and Tamhane's post hoc test. The significance level was set at 0.05; the results are given as mean ± standard deviation.

The results (mean value and standard deviation SD) are as in the following Table 2, and compositional differences between samples are highlighted in Table 3 for ease of comparison.

| Table 2. | Enamel Loss (µm) | Improved Retention (%) |
|---|---|---|
| Comparative Example A | 4.5 ± 2.6 | ---- |
| Example 1 | 3.4 ± 2.4 | 24% |
| Example 2 | 1.7 ± 4.3 | 62% |
| Example 3 | 5.0 ± 1.9 | --- |
| Example 4 | 3.9 ± 3.1 | 13% |
| Example 5 | 3.9 ± 2.3 | 13% |

**Table 3. Compositional Chart (selected ingredients, see Table 1, above, for full description)**

| Table 3 | Comparative | Example 1 | Example 2 | Example 3* | Example 4* | Example 5 |
|---|---|---|---|---|---|---|
| AmF soln. (1.4% wt.% F) | 5 | 5 | 5 | 10 | 10 | 5 |
| NaF | 0.155 | 0.155 | 0.155 | 0 | 0 | 0.155 |
| Crustacean Chitosan | 0.5 | 0 | 0 | 0 | 0 | 0 |
| Non-crustacean derived Chitosan | 0 | 0.5 | 1.0 | 0.5 | 0.5 | 0.5 |
| Silica | 22 | 22 | 22 | 22 | 17 | 27 |

| | | | | | | |
|---|---|---|---|---|---|---|
| ^{∗} not claimed | | | | | | |

Example 1 shows that at equal weight percent loading of chitosan, the dentifrice containing non-crustacean derived chitosan shows a surprising 24% improvement in enamel tissue retention when compared against the Comparative which contains crustacean chitosan.

Example 2 demonstrates another advantage of the invention. Since non-crustacean derived chitosan has a lower viscosity than crustacean chitosan, a surprisingly even greater 62% improvement in enamel tissue retention can be obtained by doubling the weight percent content of chitosan.

Example 5 demonstrates another surprising technical advantage of the invention. Example 5 is a shellfish-free dentifrice which demonstrate enamel tissue retention performance equivalent and superior to that of a known dentifrice that contains crustacean chitosan.

Examples 3 and 4 are shellfish-free and Na F-free dentifrices which demonstrate enamel tissue retention performance equivalent and superior to that of a known dentifrice that contains crustacean chitosan and Na F.

## Claims

1. A dentifrice comprising:
a source of stannous ions;
a source of fluoride ions comprising sodium fluoride and amine fluoride;
a non-crustacean derived chitosan, or pharmaceutically acceptable acid addition salt thereof; and
an abrasive system, wherein said chitosan has a molecular weight of 15 to 40 kDa, and is present in an amount of 0.4 to 1.5 weight percent based on the total composition.

2. The dentifrice of claim 1, wherein the amount of abrasive is from 12 to 30 percent by weight based on the total composition.

3. The dentifrice of any one of claim 1 or claim 2, wherein the non-crustacean derived chitosan has a molecular weight of 20 to 35 kDa.

4. The dentifrice of any foregoing claim, wherein the abrasive system is present in an amount effective to provide a PCR value of at least 50.

5. The dentifrice of claim 1, wherein the dentifrice comprises:
3000 to 4000 ppm of stannous ions;
200 to 2000 ppm, preferably 1000 to 1800 ppm fluoride ions;
0.4 to 1.5 weight percent of non-crustacean derived chitosan, or a pharmaceutically acceptable acid addition salt thereof;
17 to 27 weight percent of glycerol;
2 to 15 weight percent of sorbitol; and
an abrasive system in an amount effective to provide a PCR value of at least 50;
wherein the weight percent is based on the total weight of the dentifrice.

6. The dentifrice of claim 5, comprising:
1300 to 1500 ppm of fluoride ions;
0.5 to 1 weight percent of non-crustacean derived chitosan, or a pharmaceutically acceptable acid addition salt thereof;
3300 to 3700 ppm of stannous ions;
20 to 24 weight percent of glycerol; and
3 to 6 weight percent of sorbitol;
wherein the weight percent is based on the total weight of the dentifrice.

7. The dentifrice according to any foregoing claim, wherein the dentifrice comprises a liquid phase.

8. The dentifrice according to claim 7, wherein the stannous ions are in the liquid phase.

9. The dentifrice according to claim 7, wherein the fluoride ions are in the liquid phase.

10. The dentifrice according to claim 7, wherein the non-crustacean chitosan is in the liquid phase.

11. The dentifrice according to any of claims 8 to 10, wherein at least one of the stannous ions, the fluoride ions and the non-crustacean chitosan is dissolved in the liquid phase.

12. Dentifrice for use in a method for treating or preventing tooth demineralization in a subject in need thereof, comprising applying a dentifrice according to any one of claims 1 to 11, to an oral cavity surface of said subject.

## Patentansprüche

1. Eine Zahnpasta, umfassend:
eine Quelle für Zinnionen;
eine Quelle für Fluoridionen, umfassend Natriumfluorid und Aminfluorid;
ein von Nicht-Krustentieren abgeleitetes Chitosan oder ein pharmazeutisch akzeptables Säureadditionssalz davon; und
ein Abrasivsystem, wobei das Chitosan ein Molekulargewicht von 15 bis 40 kDa aufweist und in einer Menge von 0,4 bis 1,5 Gewichts-%, bezogen auf die Gesamtzusammensetzung, vorliegt.

2. Die Zahnpasta nach Anspruch 1, wobei die Menge an Abrasivmittel 12 bis 30 Gewichts-%, bezogen auf die Gesamtzusammensetzung, beträgt.

3. Die Zahnpasta nach einem der Ansprüche 1 oder 2, wobei das von Nicht-Krustentieren abgeleitete Chitosan ein Molekulargewicht von 20 bis 35 kDa aufweist.

4. Die Zahnpasta nach einem der vorangegangenen Ansprüche, wobei das Abrasivsystem in einer Menge vorliegt, die wirksam ist, um einen PCR-Wert von mindestens 50 bereitzustellen.

5. Die Zahnpasta nach Anspruch 1, wobei die Zahnpasta umfasst:
3000 bis 4000 ppm Zinnionen;
200 bis 2000 ppm, vorzugsweise 1000 bis 1800 ppm Fluoridionen;
0,4 bis 1,5 Gewichts-% von Nicht-Krustentieren abgeleitetes Chitosan oder ein pharmazeutisch akzeptables Säureadditionssalz davon;
17 bis 27 Gewichts-% Glycerin;
2 bis 15 Gewichts-% Sorbitol; und
ein Abrasivsystem in einer Menge, die wirksam ist, um einen PCR-Wert von mindestens 50 bereitzustellen;
wobei die Gewichts-% auf das Gesamtgewicht der Zahnpasta bezogen sind.

6. Die Zahnpasta nach Anspruch 5, umfassend:
1300 bis 1500 ppm an Fluoridionen;
0,5 bis 1 Gewichts-% von Nicht-Krustentieren abgeleitetes Chitosan oder ein pharmazeutisch akzeptables Säureadditionssalz davon;
3300 bis 3700 ppm Zinnionen;
20 bis 24 Gewichts-% Glycerin; und
3 bis 6 Gewichts-% Sorbitol;
wobei die Gewichts-% auf das Gesamtgewicht der Zahnpasta bezogen sind.

7. Die Zahnpasta nach einem vorangegangenen Anspruch, wobei die Zahnpasta eine flüssige Phase umfasst.

8. Die Zahnpasta nach Anspruch 7, wobei sich die Zinnionen in der flüssigen Phase befinden.

9. Die Zahnpasta nach Anspruch 7, wobei sich die Fluoridionen in der flüssigen Phase befinden.

10. Die Zahnpasta nach Anspruch 7, wobei sich das Nicht-Krustentieren Chitosan in der flüssigen Phase befindet.

11. Die Zahnpasta nach einem der Ansprüche 8 bis 10, wobei mindestens eines von den Zinnionen, den Fluoridionen und dem Nicht-Krustentieren Chitosan in der flüssigen Phase gelöst ist.

12. Zahnpasta zur Verwendung in einem Verfahren zur Behandlung oder Prävention von Zahndemineralisierung in einem Subjekt, das diese benötigt, umfassend das Anwenden einer Zahnpasta nach einem der Ansprüche 1 bis 11 auf eine orale Kavitätsoberfläche des Subjekts.

## Revendications

1. Dentifrice comprenant :
une source d'ions stanneux ;
une source d'ions fluorure comprenant du fluorure de sodium et du fluorure d'amine ;
un chitosane non dérivé de crustacés, ou un sel d'addition d'acide pharmaceutiquement acceptable de celui-ci ; et
un système abrasif, dans lequel ledit chitosane a un poids moléculaire de 15 à 40 kDa, et est présent en une quantité de 0,4 à 1,5 pour cent en poids par rapport à la composition totale.

2. Dentifrice selon la revendication 1, dans lequel la quantité d'abrasif est de 12 à 30 pour cent en poids par rapport à la composition totale.

3. Dentifrice selon l'une quelconque de la revendication 1 ou la revendication 2, dans lequel le chitosane non dérivé de crustacés a un poids moléculaire de 20 à 35 kDa.

4. Dentifrice selon l'une quelconque des revendications précédentes, dans lequel le système abrasif est présent en une quantité efficace pour fournir un indice PCR d'au moins 50.

5. Dentifrice selon la revendication 1, dans lequel le dentifrice comprend :
3 000 à 4 000 ppm d'ions stanneux ;
200 à 2 000 ppm, de préférence 1 000 à 1 800 ppm d'ions fluorure ;
0,4 à 1,5 % en poids de chitosane non dérivé de crustacés, ou d'un sel d'addition d'acide pharmaceutiquement acceptable de celui-ci ;
17 à 27 % en poids de glycérol ;
2 à 15 % en poids de sorbitol ; et
un système abrasif en une quantité efficace pour fournir un indice PCR d'au moins 50 ;
dans lequel le pourcentage en poids est basé sur le poids total du dentifrice.

6. Dentifrice selon la revendication 5, comprenant :
1 300 à 1 500 ppm d'ions fluorure ;
0,5 à 1 % en poids de chitosane non dérivé de crustacés, ou d'un sel d'addition d'acide pharmaceutiquement acceptable de celui-ci ;
3 300 à 3 700 ppm d'ions stanneux ;
20 à 24 % en poids de glycérol ; et
3 à 6 % en poids de sorbitol ;
dans lequel le pourcentage en poids est basé sur le poids total du dentifrice.

7. Dentifrice selon l'une quelconque des revendications précédentes, dans lequel le dentifrice comprend une phase liquide.

8. Dentifrice selon la revendication 7, dans lequel les ions stanneux sont en phase liquide.

9. Dentifrice selon la revendication 7, dans lequel les ions fluorures sont en phase liquide.

10. Dentifrice selon la revendication 7, dans lequel le chitosane non crustacé est en phase liquide.

11. Dentifrice selon l'une quelconque des revendications 8 à 10, dans lequel au moins l'un parmi les ions stanneux, les ions fluorure et le chitosane non crustacé est dissous dans la phase liquide.

12. Dentifrice pour une utilisation dans une méthode de traitement ou de prévention de la déminéralisation dentaire chez un sujet en ayant besoin, comprenant l'application d'un dentifrice selon l'une quelconque des revendications 1 à 11, à la surface de la cavité buccale dudit sujet.
